# EUROPÄISCHE PATENTSCHRIFT

(11) **EP 0 792 263 B1**
(45) Veröffentlichungstag und Bekanntmachung des Hinweises auf die Patenterteilung: **18.08.1999**
(21) Anmeldenummer: 95937870.4
(22) Anmeldetag: 06.11.1995
(51) Int. Cl.: C07C 263/10

(54) **VERFAHREN ZUR HERSTELLUNG VON ISOCYANATEN**
PROCESS FOR PREPARING ISOCYANATES
PROCEDE DE PREPARATION D'ISOCYANATES

(30) Priorität: 17.11.1994 DE 4440917; 16.06.1995 DE 19521800
(43) Veröffentlichungstag der Anmeldung: 03.09.1997
(73) Patentinhaber: BAYER AG, 51368 Leverkusen (DE)
(72) Erfinder: JOST, Klaus, D-41539 Dormagen (DE); HAMMEN, Günter, D-41569 Rommerskirchen (DE); SUNDERMANN, Rudolf, D-51375 Leverkusen (DE)
(86) Internationale Anmeldenummer: EP9504354
(87) Internationale Veröffentlichungsnummer: WO9616028

(56) Entgegenhaltungen:
- DE-A- 1 468 445
- DE-A- 2 252 068
- DE-B- 1 192 641

## Beschreibung

Die vorliegende Erfindung betrifft ein Verfahren zur Herstellung organischer Isocyanate durch Umsetzung der entsprechenden Amine mit Phosgen.

Bei der Herstellung organischer Isocyanate wird das entsprechende Amin mit Phosgen umgesetzt, wobei sich das organische Isocyanat und als Nebenprodukt Chlorwasserstoff bilden. In der Regel wird ein Überschuß an Phosgen verwendet, so daß das als Nebenprodukt anfallende Gas eine Mischung aus Chlorwasserstoff und Phosgen darstellt.

Es sind in der Literatur bereits verschiedene Methoden zur Durchführung dieses Verfahrens beschrieben. Dabei sind selbst geringe ökonomische Verbesserungen eines solch wichtigen großtechnischen Verfahrens offensichtlich von großem wirtschaftlichem Interesse.

Viele der bekannten Verfahren weisen Nachteile der Art auf, daß sie beispielsweise nur dann hohe Ausbeuten erzielen, wenn die Konzentrationen der Reaktionspartner auf niedrigem Niveau gehalten werden, oder daß sie lange Reaktions- und Verweilzeiten benötigen.

Es ist bekannt, organische Isocyanate in zwei Stufen herzustellen, indem primäre Amine mit einem Überschuß an Phosgen bei Temperaturen bis ca. 80°C umgesetzt werden und dann das carbamidsäurechlorid-haltige Produkt einer weiteren Behandlung mit Phosgen bei höheren Temperaturen zur Bildung des entsprechenden Isocyanats unterworfen wird. Üblicherweise werden im großtechnischen Maßstab die Umsetzung der Amine mit Phosgen in Phosgeniertürmen (drucklos oder auch unter Druck im Mitteldruckbereich) durchgeführt.

Es ist ferner bekannt (US 28 22 373), organische Isocyanate kontinuierlich herzustellen, indem eine Phosgenlösung in turbulenter Strömung mit einer Lösung eines organischen Amins in einem Reaktor, der in geschlossenem Kreislauf durchlaufen wird, gemischt wird. Bei diesem Verfahren kann die Lösung des Isocyanats im organischen Lösungsmittel im geschlossenen Kreislauf in den Reaktor zurückgeführt werden, um die Konzentration des Isocyanats in der Lösung zu erhöhen. Nachteilig bei diesem Verfahren ist, daß die maximale Konzentration des Isocyanats im Kreislauf nicht wesentlich über 15 % liegen und die Konzentration des organischen Amins im Lösungsmittel lediglich 5 bis 30 % betragen sollte.

In DE-A 18 11 609 werden bei der Phosgenierung Lösungen des Isocyanats in einem organischen Lösungsmittel bei Anwesenheit von mindestens 400 % Überschuß an Phosgen im Kreislauf geführt.

Weitere Phosgenierungen im Schleifenreaktor sind z.B. in JP-A 60/10774 beschrieben, in der eine isocyanathaltige Lösung umgepumpt wird, jedoch hohe Ausbeuten nur bei Aminkonzentrationen von 5 bis 10 % erzielt werden.

In DE-A 32 12 510 erfolgt zweistufig eine Phosgenierung im Schleifenreaktor in einem organischen Lösungsmittel, wobei die erste Stufe bei Normal- oder Überdrücken von bis zu 10 bar (10⁶Pa) und Temperaturen von 60 bis 100°C bei Verweilzeiten von ≥ 30 min durchgeführt wird und die zweite Stufe zum fertigen Isocyanat bei gleichem Druck und Temperaturen von 120 bis 160°C bei Verweilzeiten von ≥ 10 min durchgeführt wird.

In DE-B 11 92 641 werden Phosgenierungen in Anwesenheit des herzustellenden Isocyanates als Lösungsmittel beschrieben. Beim kontinuierlichen Betreiben des Verfahrens wird in der ersten Stufe der Umsetzung das Carbamidsäurechlorid unter ständiger Phosgenzugabe gebildet und in einer zweiten Stufe das Carbamidsäurechlorid gespalten. Nachteilig bei diesem zweistufigen Verfahren ist vor allem die lange Reaktionszeit bis zum vollständigen Auflösen der Carbamidsäure-Suspension. Bei einer kontinuierlichen Fahrweise wird das gewünschte Isocyanat als Destillat erhalten, d.h. lediglich destillierbare Isocyanate können so hergestellt werden. Auch bei der diskontinuierlichen Fahrweise werden in den Beispielen ausschließlich destillierbare Isocyanate hergestellt.

Phosgenierungen in Gegenwart eines Überschusses an organischem Isocyanat werden ebenfalls in DE-A 22 52 068 beschrieben, wobei in einem zweistufigen Verfahren aus vorerhitztem Phosgen und vorerhitztem Amin (ohne Lösungsmittel) bei überatmosphärischem Druck (ca. 100 bar) (10⁷Pa) das Carbamidsäurechlorid gebildet wird. In der zweiten Stufe bei geringerem Druck (ca. 20 bar) (2^{·}10⁶Pa) und weiterer Phosgen/Isocyanat-Zugabe wird das Carbamidsäurechlorid thermisch zum Isocyanat gespalten. Die weitere Aufarbeitung erfolgt bei einem Druck von 3 bar (3^{·}10⁵Pa) bzw. Normaldruck. Durch die vier verschiedenen Druckniveaus und die zweistufige Umsetzung ist der apparative Aufwand bei diesem Verfahren sehr hoch. Beispielhaft hergestellt werden ausschließlich destillierbare Isocyanate.

Aus DE-A 2 404 773 ist weiterhin ein Verfahren zur Herstellung von organischen Isocyanaten bekannt, bei dem in Abwesenheit eines Lösungsmittels primäre Amine mit mindestens 3 Mol Phosgen pro Aminogruppe gemischt werden, wobei die Reaktionsmischung gleichzeitig auf eine mittlere Teilchengröße von 1 bis 100 µm zerkleinert wird. Die erhaltene Suspension aus Carbaminsäurechlorid und Aminhydrochlorid in Phosgen wird bei Temperaturen von 100 bis 180°C und Drücken von 14 bis 55 bar (1,4 bis 5,5:10⁶Pa) in die entsprechenden Isocyanate überführt. Nachteilig bei diesem Verfahren ist die aufwendige und wenig betriebssichere mechanische Zerkleinerung des zunächst entstehenden Reaktionsgemisches.

Ein kontinuierliches Verfahren zur Phosgenierung unter überatmosphärischem Druck bei Temperaturen von 110 bis 195°C wurde in DE-A 1 468 445 beschrieben. Nachteilig bei diesem Verfahren ist, daß ein beträchtlicher stöchiometrischer Überschuß an Phosgen verwendet werden muß, um hohe Ausbeuten an Isocyanat zu erzielen.

Aufgabe der vorliegenden Erfindung war es daher, ein Verfahren zur Herstellung von Isocyanaten zur Verfügung zu stellen, welches es gestattet, die Umsetzung technisch einfach, d.h. in möglichst kleinen Apparaturen, in hohen Raum/Zeit-Ausbeuten durchzuführen.

Überraschend wurde gefunden, daß Phosgenierungen kontinuierlich mit einem sehr geringen technischen Aufwand und einstufig durchgeführt werden können, wenn ein Isocyanat als Lösungsmittel für Phosgen benutzt und dieses Gemisch bei Raumtemperatur in kontinuierlich zu betreibenden Reaktoren zur Phosgenierung eingesetzt wird und wenn das Amin pur oder in Lösung unter guter Vermischung der Reaktionskomponenten zugegeben wird.

Gegenstand der Erfindung ist daher ein kontinuierliches, einstufiges Verfahren zur Herstellung von Isocyanaten durch Umsetzung von entsprechenden primären Aminen mit Phosgen in Gegenwart eines Isocyanates als Lösungsmittel, das dadurch gekennzeichnet ist, daß man das gegebenenfalls in einem inerten, organischen Lösungsmittels gelöste primäre Amin mit Phosgen, das im Isocyanat zu 10 bis 60 Gew.-%, bevorzugt 30 bis 50 Gew.-%, bezogen auf die Isocyanat/Phosgen-Lösung, gelöst ist bei Temperaturen von 60 bis 180°C, bevorzugt 80 bis 150°C, und Drücken von 1 bis 30, bevorzugt 2 bis 15, zum entsprechenden Isocyanat umsetzt, wobei das molare Verhältnis von Phosgen zu eingesetztem Amin 4:1 bis 1:1, bevorzugt 3:1 bis 1,2:1 beträgt, und das als Lösungsmittel eingesetzte Isocyanat feststofffrei ist und einen Wert für hydrolysierbares Chlor von kleiner 2 %, bevorzugt kleiner 1,5, besitzt.

Als primäre Amine werden bevorzugt primäre aromatische Amine, insbesondere solche der Diaminodiphenylmethanreihe oder Gemische davon, gegebenenfalls mit deren höheren Homologen, sowie Toluylendiamin und Isomerengemische davon eingesetzt.

Das als Lösungsmittel für das Phosgen eingesetzte Isocyanat muß nicht zwangsweise das herzustellende Isocyanat sein. Es ist selbstverständlich auch möglich, andere geeignete Isocyanate als Lösungsmittel für das Phosgen einzusetzen.

Als inerte organische Lösungsmittel für die einzusetzenden primären Amine kommen insbesondere Chlorbenzol, o-Dichlorbenzol, Trichlorbenzol, Toluol, Hexan, Heptan, Octan, Xylol, Biphenyl, Essigsäureethylester, 1,2-Diacetoxyethan, 2-Butanon, Acetonitril und Sulfolan oder deren Mischungen in Betracht.

Wird das einzusetzende Amin gelöst in einem inerten, organischen Lösungsmittel eingesetzt, so beträgt die Konzentration des Amins in dem Lösungsmittel üblicherweise 10 bis 100 Gew.-%, bezogen auf das Amin/Lösungsmittel-Gemisch.

Für das erfindungsgemäße Verfahren ist es wichtig, daß das für das Phosgen eingesetzte Lösungsmittel einer bestimmten Spezifikation genügt. Bevorzugt wird das nach dem erfindungsgemäßen Verfahren hergestellte Isocyanat als Lösungsmittel eingesetzt, das dann jedoch entsprechend aufbereitet werden muß.

Das erfindungsgemäße Verfahren kann in verschiedenen Reaktoren wie Rohrreaktoren und Turm-Reaktoren, durchgeführt werden, bevorzugt in Rohrreaktoren.

Das erfindungsgemäße Verfahren kann allgemein wie folgt durchgeführt werden: Das einzusetzende primäre Amin, gegebenenfalls gelöst in einem inerten, organischen Lösungsmittel, wird mit dem im Isocyanat gelösten Phosgen unter Vermischen der beiden Reaktionskomponenten in einen geeigneten Reaktor geleitet, der bei den zuvor erwähnten Temperaturen und Drücken betrieben wird. Aus reaktionstechnischen Gründen kann es zweckmäßig sein, die Vermischung der beiden Reaktionskomponenten vor Eintritt in den Reaktor durchzuführen. Die Vermischung der Reaktionskomponenten kann dabei in üblichen Mischaggregaten, wie z.B. dynamischen und/oder statischen Mischern, durchgeführt werden. Die Verweilzeiten der Reaktionskomponenten bzw. des Reaktionsgemisches in dem Reaktor und gegebenenfalls in den Mischaggregaten beträgt üblicherweise 1 bis 30 min, bevorzugt 2 bis 15min. Die aus dem Reaktor austretende klare Lösung kann zum Abtrennen des überschüssigen Phosgen und des während der Reaktion gebildeten Chlorwasserstoffs in einer geeigneten Apparatur, gegebenenfalls unter zusätzlicher Wärmezufuhr, entspannt werden. Falls mit einem inerten, organischen Lösungsmittel gearbeitet wurde, kann ein Teil des Lösungsmittels bei der Entspannung mit abdestilliert und das überschüssige Phosgen nach Abtrennung vom Chlorwasserstoff in der Reaktion wieder verwendet werden. Das restliche inerte Lösungsmittel wird danach vom Isocyanat abgetrennt. Das wiedergewonnene inerte Lösungsmittel kann zum Lösen der primären Amine wieder zurückgeführt werden. Nach Aufbereitung kann, wie erwähnt, ein Teil des erhaltenen Isocyanats zur Herstellung der Phosgenlösung wiederverwendet werden.

Die Vorteile des kontinuierlich, einstufig zu betreibenden erfindungsgemäßen Verfahrens liegen insbesondere in den kurzen Verweilzeiten und in dem geringen apparativen Aufwand. Durch die geringen Drücke, die für das Verfahren erforderlich sind und durch den geringen Phosgen-Überschuß ist das erfindungsgemäße Verfahren auch besonders wirtschaftlich zu betreiben.

Die Erfindung soll anhand der nachfolgenden Beispiele näher erläutert werden.

### Beispiele

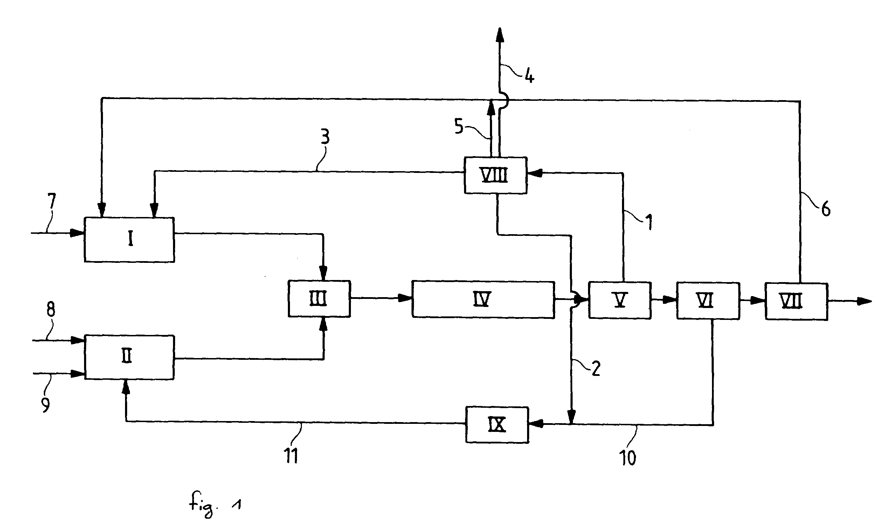

Das erfindungsgemäße Verfahren kann in einer Apparatur durchgeführt werden, wie sie aus der Figur hervorgeht.

Die nachfolgenden Versuche werden daher in einer derartigen Apparatur durchgeführt.

Amin und inertes Lösungsmittel (Chlorbenzol) für das Amin werden durch die Zuleitungen 8 und 9 in die Amin/Lösungsmittel-Vorlage II befördert. Die Temperatur dieser Vorlage wird je nach Konzentration der Aminlösung zwischen 30 und 110°C gehalten (drucklos). In der Phosgen/Isocyanat-Vorlage I wird die durch die Reaktion mit Amin verbrauchte Menge an Phosgen durch Zuspeisung durch Zuleitung 7 angeglichen. Die Temperatur in dieser ebenfalls drucklosen Vorlage liegt vorzugsweise zwischen 10 und 30°C. Bei Drücken von bevorzugterweise unter 10 bar (10⁶Pa) werden die Phosgen- und Aminlösungen in die Misch-Einheit III befördert. Dieser Misch-Einheit nachgeschaltet ist der Rohrreaktor IV, der vorzugsweise zwischen 80 und 150°C betrieben wird. Die Verweilzeiten in diesem Reaktor sind abhängig von Durchsatz und Reaktorvolumen und liegen besonders bevorzugt zwischen 1 und 5 Minuten. Die aus dem Rohrreaktor austretende klarphosgenierte Lösung wird in die Destillationseinheit V befördert. Vor der Destillationseinheit V kann eine Apparatur zur Druckhaltung eingesetzt werden. In der Destillationseinheit V wird drucklos der größte Teil an Chlorwasserstoff/Phosgen sowie gegebenenfalls inertes Lösungsmittel abgetrennt und über Leitung 1 zur Phosgenaufarbeitung VIII befördert. In VIII erfolgt die Entfernung des inerten Lösungsmittels und Chlorwasserstoffs aus dem Gemisch; das gereinigte Phosgen wird über Leitung 3 wieder in die Phosgen/Isocyanat-Vorlage I gebracht und im ebenfalls rückgeführten Isocyanat gelöst. Chlorwasserstoff wird durch Leitung 4 entfernt. Gegebenenfalls vorhandenes inertes Lösungsmittel wird durch Leitung 2 dem inerten Lösungsmittelstrom 10 und abdestilliertes Isocyanat durch Leitung 5 dem rückgeführten Isocyanatstrom 6 zugeführt. Das weitgehend phosgen-, chlorwasserstoff- und lösungsmittelfreie Isocyanat kann dann aus V in eine weitere Destillationseinheit VI gepumpt werden. Dort wird gegebenenfalls bei bevorzugten Temperaturen von 160° bis 180°C neben dem restlichen Chlorwasserstoff/Phosgen der Hauptteil des zum Lösen des Amins eingesetzten inerten Lösungsmittels (gegebenenfalls unter vermindertem Druck) abdestilliert. Das vom Isocyanat getrennte inerte Lösungsmittel wird über Leitung 10 in die Lösungsmittel-Aufarbeitung IX geleitet und dort entphosgeniert, gegebenenfalls aufdestilliert und anschließend über Leitung 11 in die Amin/Lösungsmittel-Vorlage rückgeführt. Das aus VI erhaltene Isocyanat wird in die Isocyanat-Vorlage VII gepumpt und die Menge an Isocyanat, die durch Umsetzung von Amin mit Phosgen entstand, aus dem Kreislauf ausgeschleust. Der restliche Teil an Isocyanat wird über Leitung 6 in die Phosgen/Isocyanat-Vorlage I rückgeführt.

Die Beispiele 1 bis 3 wurden in Anlehnung an DE-B 11 92 641 als Vergleichsversuche durchgeführt.

### Beispiel 1

In 300 g eines flüssigen Gemisches isomerer Diphenylmethandiisocyanate (MDI) werden zunächst bei 25°C, dann bei ca. 10°C 200 g Phosgen eingeleitet und gelöst. 100 g durch einen auf 70°C beheizten Tropftrichter flüssig gehaltenes Diaminodiphenylmethan-Isomeren-Gemisch werden unterhalb 30°C unter Rühren zugetropft, während dauernd ein Phosgenstrom in das Reaktionsgemisch eingeleitet wird. Dabei bildet sich sofort eine Suspension der Carbamidsäurechloride, die anschließend im Phosgenstrom allmählich auf 130°C erhitzt, 3,5 Stunden bei dieser Temperatur gehalten und 45 Minuten bei 145°C weiterphosgeniert werden. Nach 30-minütigem Durchleiten von Stickstoff zur Austreibung von Phosgen wird nach Abkühlen von immer noch vorhandenen geringen Mengen an Feststoff filtriert.

### Beispiel 2

In 300 g eines flüssigen MDI-Polyisocyanats, welches aus 90 % Zweikern und 10 % höherkernigen Homologen besteht, werden bei 0°C bis 10°C 214 g Phosgen gelöst und bei ca. 25°C 160 g eines flüssig gehaltenen Diaminodiphenylmethan-Isomeren/Homologen-Gemisches (90 % Zweikern und 10 % höherkernige Homologe) zugegeben und analog Beispiel 1 weiterbehandelt. Nach einer Phosgenierzeit von 4 Stunden und 45 Minuten wurde die Temperatur der Reaktionslösung auf 145°C erhöht und weitere 2 Stunden bis zum Erhalt einer klaren Lösung phosgeniert. Nach Durchleiten von Stickstoff wurden 485 g Isocyanat isoliert.

### Beispiel 3

In 300 g eines flüssigen Gemisches isomerer Toluylendiisocyanate (TDI 80/20, 80 Gew.-% 2,4- und 20 Gew.-% 2,6-Toluylendiisocyanat) werden bei 15° bis 25°C 203 g Phosgen durch Einleiten gelöst. Bei 25° bis 30°C werden dann 113 g geschmolzenes Toluylendiamin-Isomeren-Gemisch (80 Gew.-% 2,4- und 20 Gew.-% 2,6-Toluylendiamin) aus einem beheizten Tropftrichter (110°C) unter Rühren zugegeben. Währenddessen wird Phosgen weiter eingeleitet. Nach Aufheizen auf 130°C wird bei dieser Temperatur 2 Stunden und bei 145°C weitere 2 Stunden phosgeniert. Die weitere Aufarbeitung erfolgt wie in Beispiel 2 beschrieben.

### Beispiel 4

Aus einer beheizbaren 500 ml Vorlage werden pro Stunde 1,5 l einer 25.%igen Lösung eines Polymer-MDA (Diaminodiphenylmethan-Isomere und höhere Homologe in Chlorbenzol), welches einen Zweikerngehalt von ca. 50 % besitzt, mit 4 l einer 25 %igen Phosgenlösung (Phosgenüberschuß ca. 200 % auf das Amin bezogen) in rohem MDI (Diisocyanatodiphenylmethan-Isomere und höhere Homologe; Zweikerngehalt zwischen 40 und 50%) in einer Mischeinheit zusammengeführt und durch einen Rohrreaktor mit 12,5 m Länge und einem Reaktorvolumen von 157 ml, der auf 120°C temperiert ist, gefahren. Am Ende des Rohres ist ein Druckhalteventil installiert, welches auf ca. 5 bar (5^{·}10⁵Pa) eingestellt ist. Die Temperatur der Aminlösung beträgt 30 bis 40°C; die Phosgenlösung wird bei Raumtemperatur zugegeben.

Die Verweilzeit in der Mischeinheit und dem anschließenden Rohrreaktor beträgt weniger als 2 Minuten. Die Drücke sowohl amin- als auch phosgenseitig sind <10 bar (10⁶Pa). Die aus dem Rohrreaktor austretende klare Lösung wird zum Entphosgenieren in einen 2 l Ausrührtopf entspannt und bei Temperaturen zwischen 130 und 140°C von der Hauptmenge an Phosgen und Chlorwasserstoff befreit. Bei diesen Temperaturen destilliert aus dem Reaktionsgemisch bereits ein Teil des Chlorbenzols ab und dient ebenso wie in der folgenden Destillationseinheit als Strippmittel. Von dem Entphosgeniertopf wird das MDI/Chlorbenzol-Gemisch in einen Dünnschichtverdampfer (160°C, 110 bis 140 mbar) (1,1 bis 1,4^{·}10⁴Pa) zum Abtrennen des Chlorbenzols sowie der Restmengen an Phosgen und Salzsäure gepumpt.

Nach der Chlorbenzoldestillation wird die Menge an Isocyanat, die durch die Reaktion von Phosgen mit Polymer-MDA entstanden ist (ca. 470 g/h), aus dem Kreislauf ausgeschleust und der Rest in die 2,5 l Phosgen/MDI-Vorlage zurückgeführt und erneut mit Phosgen vermischt.

Das aus dem Kreislauf ausgeschleuste Produkt weist eine Viskosität von 150 mPa.s auf. Nach Temperung des Produktes wird eine Viskosität von 220 mPa.s erhalten. Der Zweikerngehalt liegt bei 43 %.

### Beispiel 5

Analog Beispiel 4 wird eine 25 %ige MDA-Lösung in Chlorbenzol (Durchsatz: 1,5 l/h, Zweikerngehalt MDA ca. 65 %) mit einer 25 %igen Phosgenlösung (Phosgenüberschuß ca. 200%) in MDI (Durchsatz 3,6 l/h) gemischt und durch einen Rohrreaktor gefahren. Der Mischeinheit nachgeschaltet ist ein 1m Rohrreaktor mit einem Volumen von 3 ml (Raumtemperatur), zwei 6 m Rohre (Volumen je 75 ml; 110° bzw. 130°C); ein 6 m Rohr mit einem Volumen von 170 ml bei 150°C und ein 2 m Rohr (Temperatur: 100°C), welches ein Volumen von 57 ml besitzt. Das Gesamtvolumen der Rohrreaktoren liegt bei ca. 380 ml, die Verweilzeit beträgt etwa 4,5 Minuten. Der Dünnschichtverdampfer zum Entfernen des restlichen Chlorbenzols wird bei 200°C und 40 mbar(4^{·}10³Pa) Druck betrieben. Das Endprodukt weist nach Destillation einen Zweikerngehalt von 39 %, einen NCO-Wert von 31,5 % und eine Viskosität von 180 mPa.s auf.

### Beispiel 6

Analog Beispiel 4 werden 110 g eines auf 70°C erhitzten reinen MDA (Durchsatz: 0,52 l/h; Zweikerngehalt MDA: 66 %) mit 1,2 kg einer 40 %igen Phosgenlösung (Phosgenüberschuß ca. 300 %) in MDI (Raumtemperatur; Durchsatz: 4,6 l/h) gemischt. Mit einer Verweilzeit von 2,6 min wird durch einen 18 m langen Rohrreaktor gefahren (Volumen: 226 ml, Temperatur: 150°C). Nach Entphosgenierung bei 160°C weist das Polymer-MDI eine Viskosität von 260 mPa.s, nach Temperung eine Viskosität von 250 mPa.s auf.

### Beispiel 7

Analog Beispiel 4 werden 675 g einer 20 %igen Toluylendiamin-Lösung (TDA-80/20-Lösung: 80 Gew.-% 2,4 und 20 Gew.-% 2,6-Toluylen-diamin-Lösung) in Chlorbenzol (Durchsatz: 1,5 l/h) mit 2,4 kg einer 25 %igen Phosgenlösung (Phosgenüberschuß ca. 200 %) in rohem Toluylendiisocyanat (TDI 80/20:2,4/2,6 TDI; Durchsatz: 5,1 l/h) gemischt. Der Rohrreaktor (12 m Länge und 150 ml Volumen) wurde bei Temperaturen von 135° und 145°C - bei einer Verweilzeit von ca. 2 min - eingesetzt. Nach Entphosgenierung und Entfernen des Chlorbenzols wurde das Isocyanat mit einer Ausbeute von ca. 98,5 % und einer Reinheit von 99,2 % (GC) destilliert.

### Beispiel 8

Analog Beispiel 4 wird ein auf 100 bis 105°C erhitztes TDA ohne Lösungsmittel (TDA 80/20, 80 Gew.-% 2,4- und 20 Gew.-% 2,6-Toluylendiamin; Durchsatz 330-380 g/h) mit einer 40 %igen Phosgenlösung (Phosgenüberschuß 200-250 %) in TDI (TDI 80/20; Durchsatz 4880 g/h) gemischt. Mit einer Verweilzeit von 2,6 min. wird das Gemisch durch einen 18 m langen Rohrreaktor gefahren (Volumen: 226 ml, Temperatur: 115°C). Nach Entphosgenierung bei 160°C wurde das Isocyanat anschließend destilliert.

## Patentansprüche

1. Kontinuierliches, einstufiges Verfahren zur Herstellung von Isocyanaten durch Umsetzung von entsprechenden primären Aminen mit Phosgen in Gegenwart eines Isocyanates als Lösungsmittel, dadurch gekennzeichnet, daß man das gegebenenfalls in einem inerten, organischen Lösungsmittel gelöste primäre Amin mit Phosgen, das im Isocyanat zu 10 bis 60 Gew.-%, bezogen auf die Isocyanat/Phosgen-Lösung, gelöst ist, bei Temperaturen von 60 bis 180 °C und Drücken von 1 bis 30 bar (10⁵ bis 3·10⁶ Pa) zum entsprechenden Isocyanat umsetzt, wobei das molare Verhältnis von Phosgen zu eingesetztem Amin 4:1 bis 1:1 beträgt und das als Lösungsmittel eingesetzte Isocyanat feststofffrei ist und einen Wert für hydrolysierbares Chlor von kleiner 2 % besitzt.

2. Verfahren gemäß Anspruch 1, dadurch gekennzeichnet, daß als Amin ein Amin der Diaminodiphenylmethanreihe oder Gemische davon, gegebenenfalls mit deren höheren Homologen, eingesetzt wird.

3. Verfahren nach Anspruch 1, dadurch gekennzeichnet, daß als aromatisches Amin Toluylendiamin und Isomerengemische davon eingesetzt werden.

4. Verfahren gemäß Ansprüche 1 bis 3, dadurch gekennzeichnet, daß die Umsetzung des Amins mit Phosgen in einem Rohrreaktor durchgeführt wird.

## Claims

1. A continuous single-step process for the preparation of isocyanates by reacting appropriate primary amines with phosgene in the presence of an isocyanate as solvent, characterised in that the primary amine optionally dissolved in an inert, organic solvent is reacted with phosgene which is dissolved in the isocyanate in a quantity of 10 to 60 wt.% based on the isocyanate/phosgene solution, at temperatures of 60 to 180°C and at pressures of 1 to 30 bar (10⁵ to 3.10⁶ Pa) to obtain the corresponding isocyanate, wherein the molar ratio of phosgene to amine used is 4:1 to 1:1 and the isocyanate used as solvent is solids-free and has a value for hydrolysable chlorine of less than 2%.

2. A process according to claim 1, characterised in that the amine used is an amine of the diaminodiphenylmethane series or mixtures thereof, optionally with higher homologues thereof.

3. A process according to claim 1, characterised in that the aromatic amine used is toluene diamine and isomer mixtures thereof.

4. A process according to claims 1 to 3, characterised in that the reaction of the amine with phosgene is carried out in a tubular reactor.

## Revendications

1. Procédé continu en un seul stade opératoire pour la préparation d'isocyanates par réaction des amines primaires correspondantes avec le phosgène en présence d'un isocyanate qui sert de solvant, caractérisé en ce que l'on fait réagir l'amine primaire éventuellement dissoute dans un solvant organique inerte avec le phosgène en solution dans l'isocyanate à une concentration de 10 à 60 % en poids, par rapport à la solution d'isocyanate/phosgène, à des températures de 60 à 180°C et sous des pressions de 1 à 30 bars (10⁵ à 3 x 10⁶Pa), ce qui donne l'isocyanate correspondant, le rapport molaire entre le phosgène et l'amine mise en oeuvre allant de 4:1 à 1:1 et l'isocyanate utilisé comme solvant étant exempt de substances solides et ayant une teneur en chlore hydrolysable inférieure à 2 %.

2. Procédé selon la revendication 1, caractérisé en ce que l'amine mise en oeuvre est une amine de la série du diaminodiphénylméthane ou un mélange de ces amines, le cas échéant avec leurs homologues supérieurs.

3. Procédé selon la revendication 1, caractérisé en ce que l'amine aromatique mise en oeuvre est la toluylènediamine ou un mélange d'isomères de la toluylènediamine.

4. Procédé selon les revendications 1 à 3, caractérisé en ce que la réaction de l'amine avec le phosgène est réalisée dans un réacteur tubulaire.
